# EUROPÄISCHE PATENTSCHRIFT

(11) **EP 3 174 589 B1**
(45) Veröffentlichungstag und Bekanntmachung des Hinweises auf die Patenterteilung: **02.10.2019**
(21) Anmeldenummer: 15753627.7
(22) Anmeldetag: 31.07.2015
(51) Int. Cl.: A61M 21/00, E04B 1/348, E04H 3/08, E04B 1/82

(54) **SYSTEM ZUR BEEINFLUSSUNG DER SINNE EINER PERSON UND EINE RAUMAUSSTATTUNG MIT EINEM SOLCHEN SYSTEM**
SYSTEM FOR INFLUENCING THE SENSES OF A PERSON AND ROOM EQUIPMENT HAVING SUCH A SYSTEM
SYSTÈME PERMETTANT D'INFLUENCER L'ESPRIT D'UNE PERSONNE ET ÉQUIPEMENT DESTINÉ À UNE PIÈCE ET MUNI DUDIT SYSTÈME

(30) Priorität: 01.08.2014 DE 102014215212
(43) Veröffentlichungstag der Anmeldung: 07.06.2017
(73) Patentinhaber: Graft Gesellschaft von Architekten mbH, 10557 Berlin (DE)
(72) Erfinder: LÜTZ, Alawi, 10117 Berlin (DE); SPIES, Claudia, 10779 Berlin (DE); WILLEMEIT, Thomas, 10115 Berlin (DE); FINKE, Annette, 10119 Berlin (DE)
(74) Vertreter: Maikowski & Ninnemann Patentanwälte Partnerschaft mbB
(86) Internationale Anmeldenummer: PCT/EP2015/067731
(87) Internationale Veröffentlichungsnummer: WO 2016/016455

(56) Entgegenhaltungen:
- EP-A2- 2 375 151
- DE-A1- 2 648 442
- DE-A1- 10 232 889
- JP-A- 2007 294 143
- US-A- 3 822 693
- US-A- 5 676 633
- US-A1- 2006 030 907
- US-A1- 2008 243 211

## Beschreibung

Die Erfindung betrifft ein System zur Messung der Wirkung von Sinneswahrnehmung innerhalb eines definierten Raums und weiterhin zur Beeinflussung der Sinne einer Person in einem solchen Raum mit den Merkmalen des Anspruchs 1 und eine Raumausstattung mit einem solchen System mit den Merkmalen des Anspruchs 10.

Reizeinflüsse beeinflussen das körperliche Wohlbefinden in vielfacher Hinsicht. Dabei ist die Erfassung bestimmter Reizwirkungen und / oder die Erzielung bestimmter Reizwirkung schwierig, da Personen vielen anderen unspezifischen Reizen ausgesetzt sind.

Systeme zur Beeinflussung der Sinne einer Person in einem Raum zur Messung bzw. Beeinflussung physiologischer Parameter der Person sind in der US 3 822 693 A und der US 2006/0030907 A1 beschrieben.

Es besteht die Aufgabe, die Beeinflussung der Sinne in einer definierten Form vorzunehmen.

Diese Aufgabe wird durch ein System mit den Merkmalen des Anspruchs 1 gelöst.

Dabei dient eine Schallschutzvorrichtung zur Abschirmung akustisch aktiver Geräte gegenüber der Person, wobei die akustisch aktiven Geräte der Messung und / oder Beeinflussung der physiologischen Parameter im Raum dienen. Die Person befindet sich dabei in einer stationären Position, d.h. sie bewegt sich nicht frei im Raum.

Zusätzlich dient eine optische Anzeigevorrichtung zur Anbietung optischer Reize und / oder Signale für die Person im Gesichtsfeld der stationären Person, wobei sich die optische Anzeigevorrichtung in mindestens einer Blickrichtung der stationär liegenden Person über einen Blickwinkel von mindestens 40° und maximal 180°, insbesondere zwischen 55° und 75° ganz insbesondere auf 70° erstreckt, wobei sich der Blickwinkel α in einer Ebene erstreckt, die sich von der Blickrichtung von oben nach unten oder von der Blickrichtung nach links und rechts erstreckt. Gerade durch die Kombination einer aktiven optischen Anzeigevorrichtung in einer bestimmten auf die Person ausgerichteten Anordnung mit einer Schallschutzvorrichtung kann die Befindlichkeit der Person gezielt gemessen und / oder beeinflusst werden.

Dabei kann es vorteilhaft sein, wenn die Neigung der stationär liegenden Person im Kopfbereich zwischen 0° und 65°, insbesondere zwischen 0° und 45° beträgt. Insbesondere in der Umgebung einer Intensivstation weisen die akustisch aktiven Geräte eine mindestens eine Infusionspumpe, mindestens eine Absaugvorrichtung, eine Dialysevorrichtung, ein Beatmungsgerät und / oder einen hämodynamischen Monitor auf.

Dabei ist es besonders vorteilhaft, die Schallschutzvorrichtung hinter dem Kopfbereich der stationär liegenden Person anzuordnen, da damit eine effektive Schallführung erreicht wird, ohne dass die Funktionalität der abgeschirmten Geräte beeinträchtigt wird. In einer vorteilhaften Ausgestaltung des Systems reduziert die insbesondere Schallschutzvorrichtung die mittlere Schallemission der aktiven akustischen Geräte im Raum auf höchstens 55 dB(A), insbesondere höchstens 40 dB(A) gemessen an der Person im Raum. Hinzu kommen ggf. noch weitere schalldämmende Maßnahmen, wie die Gestaltung eines schalldämmenden Bodens, schalldämmender Wände und Decken und / oder schalldämmender Flächen im Raum.

Ferner weist eine vorteilhafte Ausführungsform des Systems eine Schallschutzvorrichtung mit einem Mittel zum Auffangen und Dämpfen von innerhalb der Schallschutzvorrichtung abgestrahltem und / oder vom Raum in die Schallschutzvorrichtung eingestrahltem Schall auf.

Für eine umfassende optische Beeinflussung der Person ist es vorteilhaft, wenn die optische Anzeigevorrichtung sich bei der stationär liegenden Person über die Kopfposition hinaus nach hinten erstreckt.

Eine effiziente Anpassung an einen Raum, insbesondere einen Intensivpflegeraum, lässt sich erreichen, wenn die optische Anzeigevorrichtung mindestens zwei räumlich unterschiedlich orientierte Anzeigeflächen oder eine gekrümmte Anzeigefläche aufweist, wobei die optische Anzeigevorrichtung mindestens teilweise oberhalb der stationär liegenden Person in einer Ebene angeordnet ist, die insbesondere im Wesentlichen parallel zu der liegenden Position der Person orientiert ist. Damit können auf einer durchgehenden Fläche optische Reize für die Person angeboten werden.

Besonders vorteilhaft bei der Anpassung an einen Raum ist es, wenn eine zweite Anzeigefläche der optischen Anzeigevorrichtung in Blickrichtung der Person unter einem Winkel β zwischen 90° und 160°, insbesondere unter einem Winkel β zwischen 110° und 120°, ganz insbesondere mit einem Winkel β von 114° angeordnet ist, wobei der Winkel β von einer Ebene gemessen wird, die sich oberhalb und parallel zu der stationär liegenden Person erstreckt. Damit kann die Person auch in einer halbsitzenden Position optische Reize wahrnehmen.

Vorteilhafterweise ist der Übergangsbereich zwischen der ersten Anzeigefläche der optischen Anzeigevorrichtung und der zweiten Anzeigefläche der optischen Anzeigevorrichtung gerundet ausgebildet, insbesondere mit einem Krümmungsradius zwischen 20 und 40 cm. Dadurch nimmt die Person die beiden Anzeigeflächen im Wesentlichen als eine einzige Oberfläche wahr.

In einer weiteren Ausführungsform umfassen die optischen Reize und / oder Signale ausgehend von der optischen Anzeigevorrichtung Informationsdarstellungen, eine Arbeitsbeleuchtung und / oder eine biodynamische Beleuchtung. Durch die Integration von Reizanbietung und Beleuchtung kann eine platzsparende Bauweise erreicht werden und gleichzeitig eine effiziente Ansteuerung. Außerdem weist durch diese Ausgestaltung die optische Anzeigevorrichtung eine Integration in einer Fläche auf, so dass die Wahrnehmung als architektonisch wirksame Raumoberfläche, als rahmenlose Raumoberfläche, als leuchtende Decke und/oder Wand erfolgt und nicht als Gerät.

Erfindungsgemäß umfasst das System ein erstes Leuchtmittel, insbesondere ein Leuchtenfeld mit LEDs oder OLEDs, zur Funktionsbeleuchtung, Arbeitsbeleuchtung und / oder biodynamischen Beleuchtung des Raums im Bereichs der Person und ein vom ersten Leuchtmittel unabhängig steuerbares zweites Leuchtmittel, insbesondere ein LED Grid oder eine OLED Vorrichtung für die Anzeige von Mustern oder optischen Reizen, insbesondere als Unterhaltungslicht oder zur Bespielung mit visuellen Inhalten verwendet wird, wobei das erste und zweite Leuchtmittel übereinander angeordnet sind, wobei die überlagerten optischen Signale der beiden Leuchtmittel auf eine transluzente Projektionsfläche treffen, die sich im Blickfeld der Person befindet. Durch die unabhängige Steuerung kann z.B. eine cirkadiane Beleuchtung durch das erste Leuchtmittel erfolgend, wobei unabhängig davon Farbmuster oder Bilder durch das zweite Leuchtmittel angezeigt werden können.

Für ein großflächiges Anbieten der optischen Reize ist es vorteilhaft,
wenn ein Blickkegel um die Blickrichtung der stationär liegenden Person einen Öffnungswinkel von mindestens 40° und maximal 180°, insbesondere zwischen 55° und 75° ganz insbesondere 70° aufweist.

Auch können in einer vorteilhaften Ausgestaltung mit der optischen Anzeigevorrichtung optische Reize und / oder Signale für die stationär liegende Person anzeigbar sein, wobei die Reize und / oder Signale vorprogrammiert und / oder durch an der Person gemessene Daten steuerbar sind. Bei einer vorprogrammierten Steuerung können z.B. bestimmte Tageszeitmuster berücksichtigt werden. Durch eine alternative oder auch kombinierte Rückkopplung mit der Person können diese optischen Reize an die Reaktionen der Person gekoppelt werden.

Zur weiteren Schalldämmung ist es vorteilhaft, wenn die optische Anzeigevorrichtung schallschluckende Materialien aufweist.

Zur Begrenzung der Wärmebelastung des Raumes weist die optische Anzeigevorrichtung eine Kühllast von weniger als 2000 W, insbesondere weniger als 1500 W auf.

Ein besonders vorteilhafter Effekt tritt ein, wenn der Randbereich der optischen Anzeigevorrichtung mindestens teilweise eine indirekte Deckenbeleuchtung aufweist. Zusätzlich oder alternativ kann die optische Anzeigevorrichtung ein fugenloses Deckenelement darstellen.

Die Aufgabe wird auch durch eine Raumausstattung mit einem System zur Beeinflussung der Sinne einer Person in einem Raum zur Messung und / oder Beeinflussung physiologischer Parameter der Person mit den Merkmalen des Anspruchs 10 gelöst. Dabei dienen schalldämmenden Materialien in mindestens Teilen des Bodens, den Wänden und / oder der Decke des Raumes der Schaffung einer für die Person angenehmen Umgebung.

Vorteilhaft ist es, wenn mindestens zwei Systeme nach den Ansprüchen 1 bis 9 vorgesehen sind, wobei zwischen zwei stationär liegenden Personen jeweils eine Schallschutzbarriere, insbesondere als Sichtschutzbarriere angeordnet ist, wobei die Schallschutzbarriere insbesondere mindestens teilweise eine Oberfläche mit einer Holzstruktur aufweist. Die Holzstruktur bildet einen positiven visuellen Reiz für die Person.

Somit werden die Personen gegenseitig nicht gestört. Zur Schaffung eines privaten Bereiches, der insbesondere in der Umgebung einer Intensivstation wichtig ist, weist die Schallschutzbarriere auf der Seite der Person eine Vorrichtung zur Anbringung von Informationsträgern auf, auf der z.B. persönliche Gegenstände, wie Fotos oder Bilder, angeordnet werden können.

Auch ist es vorteilhaft, wenn die Schallschutzbarriere ein Mittel zum Auffangen und Dämpfen von im Inneren der Schallschutzbarriere abgestrahltem und / oder vom aus dem Raum eingestrahltem Schall aufweist.

Gerade im Bereich einer Intensivstation ist es notwendig, die Person regelmäßig zu überwachen. Zur Minimierung der Einflüsse durch diese Überwachung weist eine Ausführungsform der Raumausstattung mindestens ein Beobachtungsfenster für Beobachter außerhalb des Raums auf, wobei das mindestens eine Beobachtungsfenster zur Vermeidung von Spiegelbildern der liegenden stationären Person gegenüber der Wand des Raumes um 5 bis 10°, insbesondere 8° geneigt ist.

Eine Raumausstattung kann zwei gegenübergelegene Räume aufweisen, die über einen dazwischengeschalteten Observationsraum mit mindestens zwei Beobachtungsfenstern verbunden sind, so dass eine Beobachtung von zwei Räumen aus dem Observationsraum ermöglicht wird. Dabei ist es besonders vorteilhaft, wenn mindestens zwei Beobachtungsfenster für Beobachter außerhalb des Observationsraums - mit einer Polarisationsvorrichtung ausgestattet ist. Durch geeignete Anordnung des Polarisationswinkels kann ein freier Durchblick verhindert werden. Insbesondere kann die Polarisationsfolie jeweils in der Weise angebracht sein, dass eine Durchsicht durch zwei hintereinander liegenden Beobachtungsscheiben dadurch verhindert wird, dass die Lichttransmission durch beide Beobachtungsscheiben weniger als 98% beträgt.

Mit Vorteil ist das System der Raumausstattung eingerichtet und ausgebildet für eine liegende Person in einer Intensivpflege und / oder einem Wellnessbereich. Durch die gezielte optische und akustische Beeinflussung können z.B. die Wirkungen des Jetlags einer Person durch biodynamisches Licht gemildert werden.

Eine weitere vorteilhafte Ausgestaltung der Raumausstattung weist eine Lichtsteuerung des Raums mit folgenden Lichtparametern auf:
- Lichtstärke tags: 300 lx (morgens), 1.700 lx (mittags) und 100 lx (abends),
- Lichtstärke nachts <3 lx,
- Lichtfarbe: 2700-6500 K im Tagesverlauf angepasst an den natürlichen Verlauf,
- Lichttemperatur stufenlos regelbar in verschiedenen Szenarien mit unterschiedlichen prozentualen Anteilen und / oder
- Leuchtdichte <500 cd m⁻².
Die Bezugsgröße für die Lichtstärken ist 110 cm horizontal über dem Boden des Raums.

In einer alternativen Ausführungsform werden folgende Zielgrößen verwendet (Bezugsgröße 110 cm horizontal über dem Boden des Raums):
- Maximale Lichtstärke zwischen 1.800 und 2.800 lux, insbesondere 1.800 und 2.400 lux, ganz insbesondere zwischen 1.800 und 2.100 lux,
- Lichtfarbe: zwischen 2.700 und 6.500 K, insbesondere zwischen 3.000 und 5.000 K.

Insbesondere ist es vorteilhaft, wenn mindestens ein Lichtparameter zeitgesteuert, insbesondere dem Tagesverlauf anpassbar ist.

Eine weitere vorteilhafte Ausgestaltung der Raumausstattung weist einen schallgeschützten Raum mit mindestens einem Arbeitsplatz für Betreuungspersonal, einer Alarmstation zur Wiedergabe akustischer Alarme (Alarmweiterleitung aus dem Raum), einer Glasabwurfvorrichtung, einem Wäschelager und / oder einem Medikamentenlager auf.

Die Raumatmosphäre wird auch positiv beeinflusst, wenn die Wand im Rücken der stationär liegenden Person und / oder der Boden des Raums eine lichtreflexionsarme Oberfläche aufweist.

Ausführungsformen der Erfindung werden anhand von Abbildungen beispielhaft dargestellt. Dabei zeigen:
- Fig. 1: eine schematische Darstellung einer Ausführungsform einer Raumausstattung mit einem System zur Beeinflussung einer liegenden Person in einem Raum einer Intensivstation;
- Fig. 1A: eine Detailzeichnung mit einem geneigten Beobachtungsfenster in der Wand des Raumes gemäß Fig. 1;
- Fig. 2: eine Ansicht einer Ausführungsform des Systems zur Beeinflussung einer liegenden Person, wobei die Person flach liegt;
- Fig. 3: eine Ansicht einer Ausführungsform des Systems zur Beeinflussung einer liegenden Person, wobei die Person mit einem geneigten Kopfbereich liegt;
- Fig. 4: eine Schnittansicht durch eine Ausführungsform einer optischen Anzeigevorrichtung;
- Fig. 4A: eine perspektivische Darstellung einer Ausführungsform einer optischen Anzeigevorrichtung;
- Fig. 5: eine Ansicht einer Ausführungsform einer Schallschutzvorrichtung;
- Fig. 6: eine Ansicht einer Ausführungsform einer Schallschutzbarriere;
- Fig. 7: ein Raumplan mit Ausführungsformen eines Systems zur Beeinflussung der Sinne einer Person in einer Intensivstation.

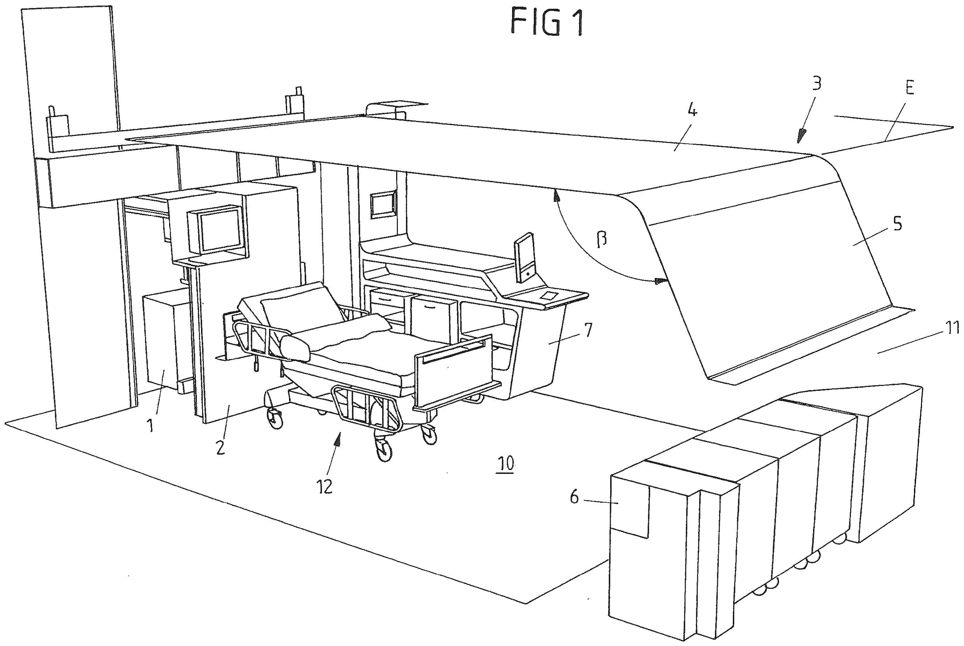

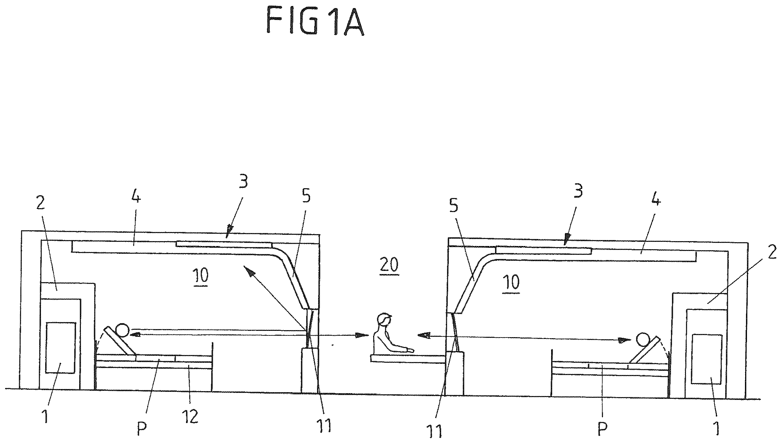

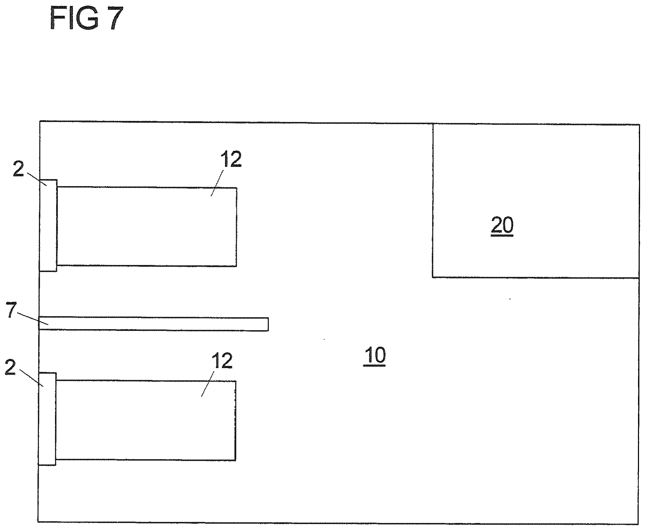

Im Folgenden werden Ausführungsformen des Systems zur Beeinflussung einer stationär liegenden Person P anhand eines oder mehrerer Räume 10 einer Intensivstation (siehe z.B. Fig. 1, 1A und 7) dargestellt. Grundsätzlich ist es aber auch möglich, die Ausführungsformen auch in der Forschung zur Erfassung von menschlichen Reaktionen oder in einem Wellnessbereich zur Entspannung einzusetzen. Eine typische Anwendung kann eine Therapie der negativen Wirkungen eines Jetlags sein.

Im Fall der Intensivstation wird die Person P als stationär liegend angenommen, d.h. die Person P befindet sich insbesondere in einem Bett 12 liegend, in dem sie sich aber grundsätzlich bewegen kann, z.B. kann die Person P sich auf die Seite drehen. Auch kann der Kopfbereich der Person P gegenüber der Horizontalen (d.h. der Ebene des Bettes) geneigt werden. Die Person P bewegt sich aber nicht frei in einem Raum 10 herum.

Die stationäre, liegende Position der Person P ermöglicht eine Ausrichtung und Einrichtung bestimmter Mittel, um die Sinne der Person P zu beeinflussen, was im Folgenden näher erläutert wird. Dabei werden optische und akustische Reize kombiniert und gezielt beeinflusst. Zunächst werden allgemeine Grundlagen des hier dargestellten Systems und der Raumausstattung mit dem System beschrieben, anschließend werden anhand von Figuren Ausführungsformen des Systems und der Raumausstattung beschrieben.

Mit einem solchen System und der Raumausstattung kann z.B. die Wahrnehmung aus der Perspektive von kritisch kranken Personen P mit dem Ziel untersucht werden, in der besonderen Ausnahmesituation eines Aufenthalts auf einer Intensivpflegestation, eine signifikante Reduktion von Stresssymptomen zur Verbesserung der gesundheitlichen Situation im Allgemeinen und zum Senken der Delirrate im Besonderen zu dokumentieren.

Bisherige Behandlungsumgebungen intensivpflichtiger Personen P beeinflussen durch eine Vielzahl von Einflussfaktoren die visuelle, akustische Wahrnehmung, die räumliche und zeitliche Orientierung. Dabei können insbesondere Phänomene von Hilflosigkeit bis zu Panik, insbesondere als Angst- und Stressfaktoren identifiziert werden, die auf die Personen P wirken. Die bisherige Praxis einer vermehrten Gabe von Schmerzmitteln, bzw. der Sedierung der Personen P führen neben einem festzustellenden verlangsamten Heilungsverlauf insbesondere auch zu einer erhöhten Delirrate.

Eine Verbesserung der Umgebung der Personen P erscheint wünschenswert. Dabei ist die Raumausstattung und insbesondere die Wirkung aller auf die Wahrnehmung der Gesundheit der zu behandelnden Patienten in dem Sinn zu verbessern, dass die Umgebung angst- und stressmindernd wirkt, so dass die Gabe von Schmerzmitteln bzw. Sedierung reduziert und somit Heilungsverläufe optimiert gefördert und die Delirrate reduziert werden kann.

Durch gezielten Ersatz des fehlenden Tageslichts, durch Beeinflussung der Tag- und Nachtrhythmen und / die Minimierung negativer Effekte durch erhöhte Lärmbelastung können die Personen P durch das System gezielt beeinflusst werden und die Wirkungen auf die Person P gleich miterfasst werden.

Eine umfassende Untersuchung gleichzeitig wirkender Wahrnehmung ist in bisherigen Intensivzimmer-Umgebungen bisher nicht möglich, da sich die konventionelle Zimmerarchitektur weitestgehend auf die Erfüllung funktionaler Kriterien einer erforderlichen medizinischen Behandlung beschränken und einflussmächtige Wahrnehmungsphänomene aus Patientensicht außer Acht lassen. Zur optimalen Betrachtung und Beobachtung solcher Wahrnehmungsphänomene und möglicher Einflussnahme auf diese zur Förderung des Heilungsverlaufes sind Zimmer- und Medienarchitekturen unter Verwendung des hier dargestellten Systems zur Beeinflussung der Person P entwickelt worden.

Um diese Phänomene in Ihrer Gesamtheit zu untersuchen, wird hier in einer Ausführungsform ein Versuchsaufbau beschrieben, der Einzelfaktoren berücksichtigt und auf diese Einfluss nehmen kann. Der Versuchsaufbau kann in gleicher oder abgewandelter Form in realen Intensivstationen eingesetzt werden.

Die Ausführungsformen beruhen auf den typischen Anordnungen und Erfordernissen eines intensivmedizinischen Raums 10. Ferner wird eine betreuungs- und pflegeunabhängige Raumkonfiguration entwickelt, die akustischen und visuelle Wahrnehmungsparameter mit einbezieht, so dass der Heilungsverlauf positiv beeinflussbar ist und das Auftreten von durch Angst und Stress verbundenen Krankheitsbildern minimiert wird.

Wichtige Einflussparameter sind insbesondere die Raumakustik, Lärm, Verkehr auf der Intensivstation, subjektives Empfinden der Hilflosigkeit und Abhängigkeit, subjektives Empfinden der Raumtemperatur, zeitliche und räumliche Orientierung und insbesondere auch visuelle Reize oder Reizarmut im Bereich des Blickkegels (auch "view cone" genannt) der stationär liegenden Person P.

Diese Einflussparameter haben Einfluss auf das Entstehen von Angst, Stress, subjektivem Schmerzempfinden und im Weiteren einen gehemmten Heilungsverlauf und das verstärkt diagnostizierte Auftreten von Delir und Halluzinationen haben.

Da diese Krankheitsbilder nicht nur als diagnostizierte Einschränkung selber von Bedeutung sind, sondern insbesondere in postoperativen Ausnahmezustand einen Heilungsverlauf verlangsamen, hemmen oder ganz verhindern können, kommt allen nachweisbar identifizierbaren Einflussfaktoren eine besondere Relevanz zu. Das System zur Beeinflussung der Sinne der Person P kann u.a. dazu dienen, Einzelheiten der Einflussparameter zu identifizieren, sie zu benennen und ggf. Strategien zu entwickeln, wie ihnen begegnet werden kann. Das System kann aber auch in der in der regulären Therapie in Intensivstationen oder regulären Krankenstationen eingesetzt werden.

Zusätzlich zur erforderlichen medizinischen Betreuung auf Intensivstationen wird ein System beschrieben, mit dem eine Beobachtung von Personen P vorgenommen werden kann.

Die Konzentration darauf, was die Person P hört und sieht, ist der Ausgangspunkt für einen Paradigmenwechsel in der Intensivmedizinarchitektur weg vom funktional technischen Aufbau mit im Rahmen der medizinischen Betreuung notwendigen Gerätschaften und Versorgungsleitungen hin zu einer aus Patientensicht beschreibbar "erlebten" Szenografie. Diese soll aus medizinischer Sicht das subjektive Empfinden der Patienten vorhersehbar positiv beeinflussbar machen und sich so messbar auf den Heilungsverlauf der Patienten auswirken. Die Verringerung von Stress durch Reduktion von Stressfaktoren und der Aufbau als Wahrnehmungsmaschine für Patienten, die zu Ablenkung, Entspannung oder Unterhaltung genutzt werden kann, führt zu der These einer tatsächlichen Beeinflussbarkeit des physischen Zustands durch Raumkonfiguration und Architektur in neuartigem Versuchsaufbau.

Für die stationär liegende Person P ist im Rahmen der Intensivstation das Gesichtsfeld der Person P relativ genau definierbar. Nicht nur Befragungen sondern auch Messungen können kontrolliert angestellt werden und in der Betrachtung verschiedener Personen P eine Vergleichbarkeit hergestellt werden. Darüber hinaus scheinen im Ausnahmezustand eines postoperativen stationären Aufenthalts auch die Einflussfaktoren gemeinhin beiläufiger Einflüsse wie fehlendes Tageslicht, Unruhe während des Schlafes, ungewohnte Akustik und Umgebung, unbekannte Umgebung und Gefahr der Desorientierung einen besonderen Einfluss auf den Heilungsverlauf zu haben, der hier aber im Vordergrund jeder medizinischen Betrachtung steht. Dies lässt darüber hinaus auch die Annahme zu, dass sich im Rahmen der Wahrnehmungs- und Delirforschung Erkenntnisse zur Wirkung von Räumen und Raumumgebungen im Allgemeinen ableiten lassen.

Patienten auf Intensivstation leiden regelhaft unter Störungen des Schlaf-Wach-Rhythmus die mit einer schlechten Schlafqualität einhergehen. Licht ist der wichtigste Taktgeber zur Aufrechterhaltung des beim Menschen physiologischen Tag-Nacht-Rhythmus. Dieser Rhythmus wird durch das epiphysiale Hormon Melatonin gesteuert. Störungen des Tag-Nacht-Rhythmus sind daher in der Regel durch eine Dysregulation des physiologischen Profils der Melatoninausschüttung gekennzeichnet. Die Sekretion des epiphysalen Hormons Melatonin, welches aus der biogenen Aminosäure Tryptophan synthetisiert wird, ist abhängig von der Lichtexposition. Neben der Erkrankung selbst sowie den Medikamenten die der Patient während seiner intensivstationären Behandlung erhält, können daher auch inadäquate Lichtbedingungen in den Behandlungszimmern, Schlafstörungen auslösen bzw. mit unterhalten die in der Folge Organfunktionsstörungen des Gehirns mit bedingen und somit einen nicht nur akut sondern auch langfristig negativen Effekt auf das Behandlungsergebnis des Patienten haben.

Im Rahmen der hier dargestellten Ausführungsformen geht es um eine Schaffung einer Raumatmosphäre die
- dem behandelten Patienten im Raum Angst nimmt wodurch sich sedierende Medikamente einsparen lassen, die in der Folge einen positiven Einfluss auf den allgemeinen Heilverlauf der Patienten haben könnte.
- dem behandelten Patienten im Raum tageszeitliche Orientierungshilfen bietet, wodurch akute Delirien und Verwirrtheitszustände von Patienten reduziert werden können, die in der Folge einen positiven Einfluss auf den allgemeinen Heilverlauf der Patienten haben könnte.
- Arbeitsprozesse für das Personal so optimiert, dass auf der einen Seite ein effektiveres und zeiteffizienteres, sicheres Arbeiten möglich und auf der anderen Seite die übrigen hier festgelegten Ziele mit unterstützt werden.

Zur Entwicklung des räumlichen Versuchsaufbaus wurden zunächst die einzelnen Einflussparameter identifiziert die einen Einfluss auf die Wahrnehmung der Person P ermittelt.

### Akustische Wahrnehmung

Ausgehend von der räumlichen Positionierung der Person P im Raum 10 wurden zunächst Informationen zu den bestehenden Schallquellen aufgenommen und auf die definierten Zielgrößen hin optimiert. Definierte Zielgrößen für Ausführungsformen des Systems sind:
- Tag: Lärmreduktion: Lärmpegel <55 dB(A)
- Nacht: Lärmreduktion: Lärmpegel <40 dB(A)
- Spitzenpegel >85 dB(A) vermeiden
- kontinuierliche (>30 min) Spitzenpegel >70 dB(A) vermeiden

Diese - und auch alle vergleichbaren Werte im Folgenden - sind als zeitliche und / oder räumliche Mittelwerte zu verstehen.

Durch die her dargestellten Ausführungsformen können die mittleren Schallpegel in den Behandlungszimmern um bis zu 6 dB reduziert werden, was einer Reduktion der Lautstärke auf das 0,66fache entspricht. Zudem konnten wir den Anteil an maximalen Schallpegeln von >60 dB in der Nacht, in den Behandlungszimmern um mehr als die Hälfte reduzieren (27-30%). Dabei können insbesondere die Häufigkeit von nächtlichen, durch Behandelnde verursachte Schallpegelspitzen, in den Behandlungszimmern reduziert werden. Der in einer Ausführungsform zwischen den beiden Räumen geschaffene Observationsraum 20 scheint hierbei eine entscheidende Rolle zu spielen.

In einer Ausführungsform sind für eine liegende Person jeweils zwei Lautsprecher mit gleichem Abstand zu den Ohren der Person vorgesehen.

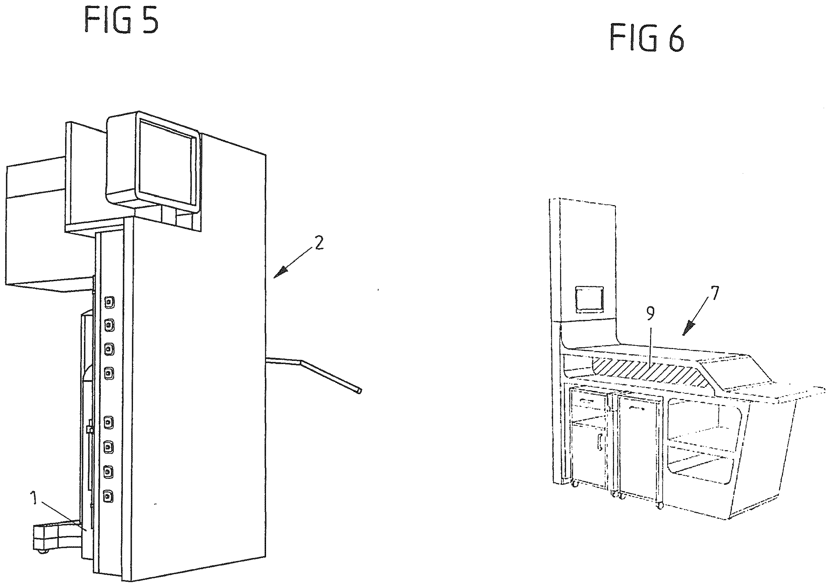

Dies kann z.B. in einfacher Weise dadurch erreicht werden, indem die Lautsprecher rechts und links der Person P in Schallschutzbarrieren eingebaut werden, die z.B. in Fig. 6 dargestellt sind.

Durch die symmetrische Beschallung wird ein beruhigender Effekt erreicht. Die Lautsprecher weisen eine entsprechende Richtcharakteristik zur Beschallung der definierten Patientenzone und Vermeidung von Diffusschall (Schutz des Nachbarpatienten) auf.

Dabei kann in einer Ausführungsform eine individuelle Steuerung vorgesehen sein, die zu einer Beruhigung und kognitiven Stimulanz der Person P führt. Die Ausführung im Einzelzimmer ermöglicht in einer besonderen Ausführung die Untersuchung der (beruhigenden) Wirkung verschiedener Beschallungsmuster.

Auch kann eine Vorrichtung zur aktiven Lärmkompensation vorgesehen sein, bei der lärmender, unerwünschter Schall durch destruktive Interferenz ausgelöscht oder zumindest erträglicher gemacht wird.

Mit Hilfe von Lautsprechern mittels einer Klanglandschaft kann eine gesundheitsfördernde Raumatmosphäre generiert werden, die durch akustische Überlagerung der gegebenen Umgebungsgeräusche eine beruhigende, persönlich adaptierbare und somit vertraute Umgebung schafft. Geräuschquellen mit negativ empfundener Geräuschcharakteristik (Rauschen Beatmungsgerät) können somit weniger präsent wahrgenommen werden.

### Visuelle Wahrnehmung

Eine Optimierung der Lichtverhältnisse auf der Intensivstation verbessert die Schlafqualität der Personen P, wobei auch signifikant weniger Schmerzmedikamente appliziert werden müssen und weniger depressive Symptome auftreten.

Wichtige Parameter der Beleuchtung sind dabei die Blendung, die Lichtstärke, die Lichtfarbe und / oder die zeitliche Abfolge dieser Parameter über den Tagesverlauf. Definierte Zielgrößen sind dabei (Bezugsgröße 110 cm horizontal über dem Boden des Raums (10)):
- Lichtstärke tags: 300 lx anfangs (morgens), 1.700 lx (mittags) und 100 lx abends , d.h. es liegt eine cirkadiane (biodynamische) Lichtsteuerung vor.
- Lichtstärke nachts <3 Ix.
- Lichtfarbe: 2700-6500 K im Tagesverlauf angepasst an den natürlichen Verlauf.
- Lichttemperatur stufenlos regelbar in verschiedenen Szenarien mit unterschiedlichen prozentualen Anteilen.
- Blendverhalten, Leuchtdichte so niedrig wie möglich halten Beleuchtungsfläche ausreizen. <500 cd m⁻².

In einer alternativen Ausführungsform werden folgende Zielgrößen verwendet (Bezugsgröße 110 cm horizontal über dem Boden des Raums (10)):
- Maximale Lichtstärke zwischen 1.800 und 2.800 lux, insbesondere 1.800 und 2.400 lux, ganz insbesondere zwischen 1.800 und 2.100 lux,
- Lichtfarbe: zwischen 2.700 und 6.500 K, insbesondere zwischen 3.000 und 5.000 K.

Dabei liegen die Lichtstärken über der für die Melatoninsuppression erforderlichen Schwelle. Auch werden technische Möglichkeiten der automatisierten Anpassung von Licht-Farbtemperatur zur tageszeitlichen Rhythmisierung der Patienten. Dabei besteht eine ausreichende Beleuchtungsstärke bei gleichzeitigem Einhalten der absoluten Blendtoleranz von 10.000 cd/m² gerade auch bei maximaler Beleuchtungsstärke in Blickrichtung der Person (P) zum Leuchtmittel.

Mit Ausführungsformen der Erfindung ist es gerade möglich, gezielt Untersuchungen durchzuführen, um bestimmte Parameterkombinationen zu ermitteln. Die Ausführungsformen sind dabei flexibel genug, entsprechende Zielgrößen nachträglich einstellen zu können und das ausreichende Beleuchtungsstärken erreicht werden.

Auch kann mit den Ausführungsformen ermittelt werden, wie hoch die Lichtstärke liegen muss um die für die Melatoninsuppression erforderliche Schwelle zu überschreiten und welche Bestrahlungsdauer der höchsten Lichtstärke für die Beschriebenen Effekte zielführend ist. Die über den Tagesverlauf eingesetzten Lichtstärken erreichen im Tagesmittel Blendwerte von 400 bis 600 cd/m², insbesondere um 500 cd/m². Die Mittelung wird dabei z.B. über das Blickfeld der Person (P) und die Zeit vorgenommen.

Wenn eine der Ausführungsformen zu Forschungszwecken eingesetzt wird, so können z.B. folgende Maßnahmen vorgenommen werden:
- Schlafstadien-Bestimmung mittels Elektroenzephalographie (Polysomographie)
- Bestimmungen von Melatonin, Kortisol und spezifischen Uhrengenen (molekulagenetische Messungen) im Blut zur Abbildung (von Störungen) der zirkadianen Rhythmik.
- Durchführung der geplanten kognitiven Testbatterien und Fragebögen zur Lebensqualität sowie posttraumatischen Belastungsstörungen im Rahmen eines Follow-Up's, 3 und 6 Monate nach Entlassung von der Intensivstation.

Durch die hier skizzierten Ergänzungen und Anpassungen des ursprünglichen Studienprotokolls, kann letztendlich eine sehr viel differenziertere wissenschaftliche Aussage über die Wirksamkeit der entwickelten Innovationen gemacht werden.

Die akustischen und visuellen Reize haben eine besondere Bedeutung. Des Weiteren gibt es noch eine Reihe weiterer Einflussgrößen.

### Raumklima

Definierte Zielgrößen sind dabei:
- Optimierung Lüftungsströmung auf die Position der Person P
- Optimierung Luftfilter
- Gestaltung keimfreier Umgebung
- Einsatz von warmen Materialien wie z.B. Holzwerkstoffe

Im Folgenden werden Maßnahmen beschrieben, die Einzeln oder in Kombination auch dazu verwendet werden, das Umfeld der Person P zu verbessern.

### Maßnahmen betreffend die Akustik

- Akustisches Verstecken der akustischen aktiven medizinischen Geräte 1 vor der Person P durch eine Schallschutzvorrichtung 2, insbesondere eine Rückwand hinter der Person P. Dabei werden Geräusche der akustischen aktiven medizinischen Geräte 1 wie z.B. Piepgeräusche, Alarmsignale der Infusoren, Geräusche von Überwachungsmonitoren, Beatmungsgeräten oder Dialysegeräte Patienten gedämpft. Durch die Positionierung z.B. des Beatmungsgerätes oder der Infusoren hinter der Schallschutzvorrichtung (oder im Schallschatten) am Kopfende des Bettes 12 befindet sich die Person P nicht im Feld des Direktschalls. Insbesondere kann eine dreiseitige Einhausung den direkten Lärmeintrag auf die Person P abschirmen. Auch andere medizinischen Kleingeräte, wie z.B. Absauggeräte, Gasanschlüsse und sonstiger Anschlüsse können seitlich oder hinter der Schallschutzvorrichtung 2 am Kopfende des Bettes 12 angeordnet sein, so dass die Person P nicht im Feld des Direktschalls dieser Geräte 2 liegt. Auch wird der Schalleinfluss minimiert, der durch Arbeiten an den Anschlüssen oder Geräten 2 anfällt.
- Mit der Positionierung einer Arbeitsebene auf den akustisch abgeschirmten Bereich räumlich ca. 30 cm bis 90 cm hinter der liegenden Person P wird eine geringere Störung der Person P durch Arbeitsabläufe des Pflegepersonals erreicht.
- Mit einer Positionierung einer Schallschutzbarriere 7 zwischen zwei Betten 12 mit einer Höhe von z.B. 120 cm im Bereich des Kopfendes des Bettes 12 wird Direktschall abgeschirmt. Vor- und Rücksprünge in der Schallschutzbarriere übernehmen die Funktion, Direktschall zu zerstreuen und damit Gesprächsinhalte für den Nachbarpatienten unverständlich zu halten. Es wird zudem eine private Ebene in Augenhöhe der Person P zur positiven Überlagerung / Ablenkung geschaffen.
- Rauschen der Lüftung wird gedämpft.
- Vermeidung von Arbeitsgeräuschen und Gespräche des Pflegepersonals am Patientenbett, am Nachbarpatienten und durch den Korridor.
- Vermeidung schallharter Oberflächen der Möblierung. Reduzierung des Umgebungsgeräuschpegels durch Pflegepersonal durch angepasstes Raumlayout mit mittig angeordnetem Observationsraum 20 (indirekte Patientenüberwachung). Im Gegensatz zu einen bekannten Patientenraum, in denen alle Arbeiten des Pflegepersonals innerhalb des Raums 10 stattfinden, wird die räumliche Struktur so verändert, das ein abgeschlossener Observationsraum 20 zwischen zwei Patientenräumen 10 geschaffen wird, in dem Arbeiten wie Dokumentation, Materialnachfüllen, Diagnose, Diskussion zur Visite etc. stattfinden können. In dem Observationsraum 20 werden auch Alarme der medizinischen Geräte aufgeschaltet, sodass diese nicht störend im Patientenzimmer 10 auftreten. Hierdurch werden auch Alarmgeräusche, Arbeitsgeräusche, Nebengeräusche des Pflegepersonals, wie Gespräche etc. akustisch ausgeblendet.
- Weitere Reduzierung der Alarmgeräusche mit aufgeschalteten Alarmen in Observationsraum 20.
- Reduzierung des Umgebungsgeräuschpegels durch Einsatz selbstschließender Türsysteme.
- Senken des Schallpegels und der Nachhallzeit im Raum 10 durch schalldämmende Materialien im Deckenbereich und Einsatz von schallweicher Bespannung der Deckenelemente, einschließlich mindestens teilweise der optischen Anzeigevorrichtung 3.
- Reduzierung des Arbeitsgeräuschpegels durch mobiles Mobiliar.
- Reduzierung der wahrgenommenen Geräusche des Nachbarpatienten durch Schallschutzbarriere 7 zwischen beiden Betten 12.
- Reduzierung des Geräuschpegels durch Einsatz von Holzwerkstoffmaterialien bzw. Mineralwerkstoffplatten im Gegensatz zu Edelstahlmaterialien. (Tür- oder Schubladengeräusche / Schwingungsverhalten / niedrigerer Frequenzbereich).
- Einsatz von schweren Holzwerkstoffplatten und Mineralwerkstoffplatten zur Schallabschirmung.

### Maßnahmen betreffend die visuelle Wahrnehmung

Die Gestaltung der sichtbaren Oberfläche des Raums 10 als räumlich kontrollierte Figur ermöglicht die Kontrolle der visuellen Wahrnehmung der Person P z.B. durch eine programmierbare Bildebene und / oder bildgebende Elemente auf der Oberfläche einer optischen Anzeigevorrichtung 3. Unter Berücksichtigung des tatsächlichen Gesichtsfelds der stationär liegenden Person P kann hier als zentrale Einheit ein großformatiges Deckenelement als optische Anzeigevorrichtung 3 verwendet werden, mit der visuelle Inhalte kontrolliert angezeigt werden können, aber auch optimierte Beleuchtungselemente zu integriert werden können.

Wie im Folgenden noch dargestellt wird, kann die Anordnung der optischen Anzeigevorrichtung 3 nicht nur an horizontalen Teilen der Decke erfolgen.

Die Verwendung von natürlichen Oberflächen / Holzmaserungen hat nicht nur einen akustischen Effekts, sondern auch einen visuellen.

Im Folgenden werden einige Merkmale der optischen Anzeigevorrichtung 3 aufgeführt, die einzeln oder kombiniert eingesetzt werden können:
- Überlagerung steuerbarer Beleuchtungselemente mit einem zweiten Leuchtmittel 31, insbesondere ein LED Grid zur kontrollierten Wiedergabe von optischen Mustern oder Inhalten für die Person P.
- Programmierung des Verlaufs von Lichtstärke und -farbe in zeitlicher Abhängigkeit zur Tages und / oder jahreszeitlicher Einordnung.
- Optimierter Einbau der Leuchtmittel 30, 31 in Bezug auf den Blickwinkel oder das Gesichtsfeld der Person P.
- Integrieren von mehr als einer Beleuchtungsfunktion in der optischen Anzeigevorrichtung 3, insbesondere das erste Leuchtmittel 30: Grundbeleuchtung, zirkadianes Licht, biodynamisches Licht und Arbeitslicht.
- Schichtweiser Aufbau der optischen Anzeigevorrichtung 3 mit unterschiedlichen Leuchtmitteln 30, 31 für die Funktionen Biodynamisches Licht Funktionslicht / Arbeitslicht / Atmosphärisches Licht in einer fugenlosen Fläche als integrale Raumkomponente.
- Die über den Betten 12 großflächig angebrachte optische Anzeigevorrichtung 3 weist eine schallweiche Bespannung 32 auf, die auch eine Projektionsfläche bildet.
- Jedes einzelne Bett 12 wird mithilfe einer seitlich flankierenden Schallschutzbarriere 7, der rückseitigen Schallschutzvorrichtung 2 und der optischen Anzeigevorrichtung 3, die auch an der gegenüberliegenden Bettwand bis auf 150 cm heruntergeführt akustisch eingefasst. Dies bedeutet, dass die optische Anzeigevorrichtung 3 insbesondere mit der Schallschutzvorrichtung 2 zusammenwirkt, um eine definierte Umgebung für die Person P zu schaffen.
- Reduzierung des Blendempfindens durch warmweiße LED in Vouten seitlich der optischen Anzeigevorrichtung 3.

Die Raumwahrnehmung des realen Raumes 10 und der medial bespielten Oberflächen der optischen Anzeigevorrichtung 3 bilden komplementären Pole eines atmosphärischen und szenografischen Konzeptes, welches der Wahrnehmungsforschung unter Licht-, Lärm- und Temperatureinwirkung dient.

Raumbedingte Stressfaktoren optischer Art sollen messbar reduziert werden. Zur optimalen Wirkung der optischen Anzeigevorrichtung 3 und deren Szenografie ist es notwendig, eine Reihe von allgegenwärtigen Störfaktoren im Intensivzimmer, d.h. im Raum 10 auszuschließen oder zumindest zu mindern.

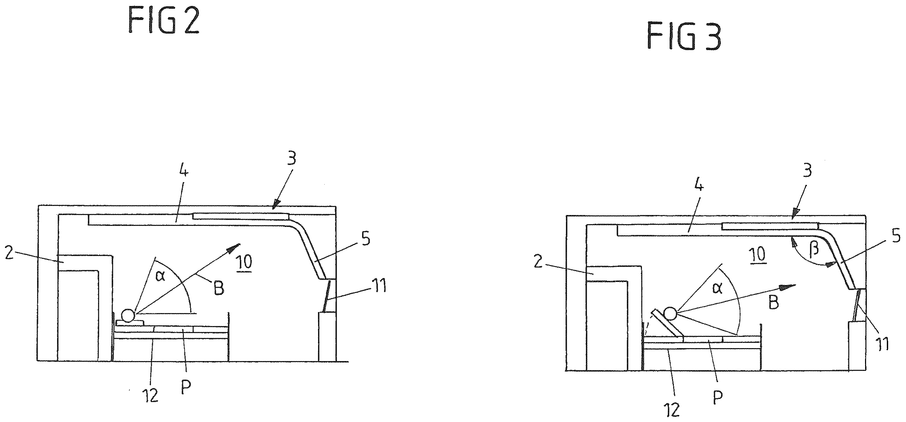

In Fig. 1 ist eine perspektivische Teilansicht eines Raums 10 in einer Intensivstation dargestellt. Die Person P, die hier nicht dargestellt ist, liegt typischerweise im Bett 12 und liegt flach oder mit dem Oberkörper bis zu 45° gegenüber der Ebene des Bettes 12 geneigt. Damit kann die Blickrichtung B - bezogen auf die Ebene des Bettes 12 - von senkrecht nach oben bis etwa zu 45° gegenüber der Ebene des Bettes 12 geneigt sein (siehe Fig. 2 und 3). Somit überstreicht der Blickkegel typischerweise einen Bereich der sich von der Fläche oberhalb des Bettes 12 bis zu einen Bereich vor dem Bett 12 erstreckt.

In diesem Bereich ist eine optische Anzeigevorrichtung 3 angeordnet, die hier zwei Anzeigeflächen 4, 5 aufweist, die in Blickrichtung B der Person P unter einem Winkel β zwischen 90° und 160°, insbesondere unter einem Winkel β zwischen 110° und 120°, ganz insbesondere mit einem Winkel β von 114° angeordnet sind, wobei der Winkel β von einer Ebene E gemessen wird, die sich oberhalb und parallel zu der stationär liegenden Person P erstreckt. Der Übergangsbereich zwischen den beiden Anzeigeflächen 4, 5 weist einen Krümmungsradius von 20 bis 40 cm auf.

Die optische Anzeigevorrichtung 3, d.h. beide Anzeigeflächen 4, 5 sind hier 2,44 m breit, die gesamte Länge der beiden Anzeigeflächen 4, 5 beträgt hier in der Abwicklung 5 m, so dass die horizontale Anzeigefläche 4 noch über den Kopfbereich der Person P hinausgeht. Grundsätzlich kann die Größe der optischen Anzeigevorrichtung 3 der Person P angepasst werden.

Für die stationär liegende Person P füllt die optische Anzeigevorrichtung 3 einen Blickbereich von mindestens 70° im Wesentlichen aus, so dass damit gezielt die optischen Sinne der Person P beeinflusst werden können.

In anderen Ausführungsformen können auch andere Abmessungen und Winkelmaße gewählt werden.

Die Ausführungsform des Systems zur Beeinflussung der Sinne ist aber nicht nur auf optischen Reize ausgerichtet, sondern es trägt in Kombination auch der Beeinflussung der akustischen Reize Rechnung. Beide Maßnahmen gemeinsam und in Wechselwirkung bilden das System.

Dazu ist im Raum 10 hinter dem Bett 12 eine Schallschutzvorrichtung 2 angeordnet, hinter der akustisch aktive Geräte 1 angeordnet und / oder in diese integriert sind. Hier handelt es sich um medizinische Mess- und Therapiegeräte, wie z.B. Beatmungsgeräte, Infusoren oder Dialysegeräte. Diese Geräte strahlen in einem weiten Spektrum akustische Signale aus, die das Wohlbefinden der Person P negativ beeinflussen.

Dieses System mit den beschriebenen optisch und akustisch Vorrichtungen ist in einem Raum 10 angeordnet, wobei insbesondere auch mehr als ein System vorgesehen sehen sein kann. Diese bilden dann eine Raumausstattung.

In Fig. 1 ist erkennbar, dass eine Schallschutzbarriere 7 das Bett 12 von einem anderen, hier nicht dargestellten Bett 12 mit einer weiteren Person P trennt. Dies wird im Folgenden noch näher erläutert werden.

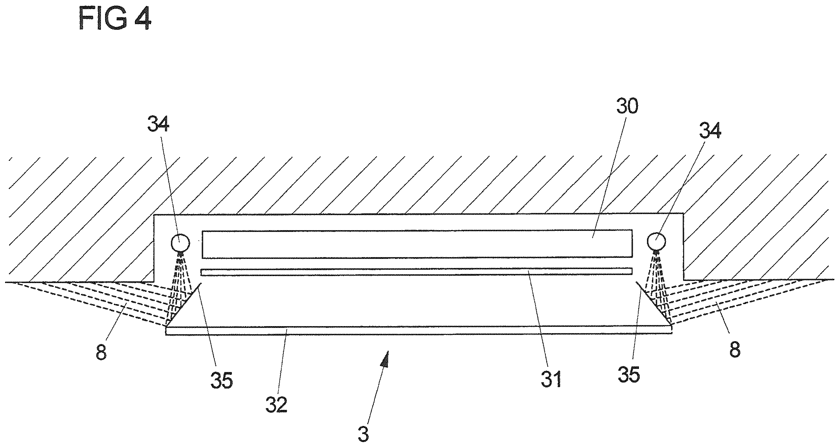

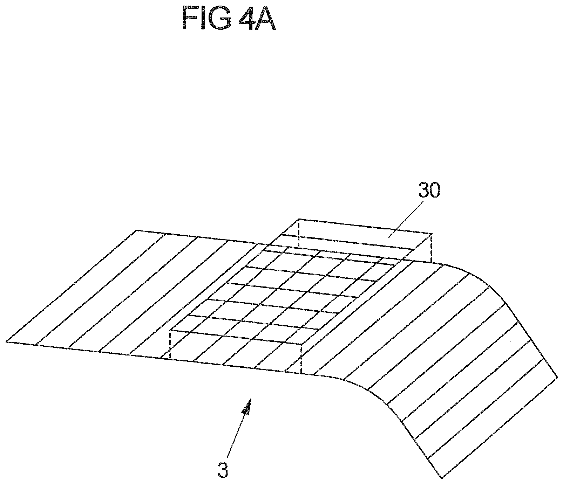

Auch ist in dem Raum 10 eine Lichtsteuerung 6 angeordnet (hier nur beispielhaft dargestellt), mit der das Licht im Raum 10 insbesondere dem Tagesrhythmus angepasst werden kann, wie dies oben beschrieben worden ist. Die optische Anzeigevorrichtung 3 (siehe Fig. 4) kann neben der Darstellung von optischen Inhalten auch der Raumbeleuchtung dienen.

Mit den im Raum 10 befindlichen diagnostischen Geräten kann die Wirkung der der Reize auf die Person P registriert werden. Damit kann ggf. auch eine Rückkopplung realisiert werden, in dem die akustischen und optischen Reize an die jeweiligen Reaktionen der Person P angepasst werden.

In Fig. 1A ist eine Schnittansicht durch einen Raum 10 dargestellt, bei dem zwei Betten 12 auf gegenüberliegenden Seiten eines schallgeschützten Raumes 20 (Observationsraum) angeordnet sind. Die Raumausstattung der Räume 10 - insbesondere die Schallschutzvorrichtung 2 und die optische Anzeigevorrichtung 3 entspricht dabei der Ausführungsform in Fig. 1, so dass auf die obige Beschreibung Bezug genommen werden kann.

Die Personen P liegen hier mit geneigtem Oberkörper (ca. 35 bis 40° gegenüber der Ebene des Bettes 12) in den Betten 12, so dass die Blickrichtung B auf ein Beobachtungsfenster 11 zeigt. Um irritierende Spiegelungen zu vermeiden, insbesondere auch die Betrachtung des eigenen Spiegelbildes, sind die Scheiben des Beobachtungsfensters 11 um 8° gegenüber der Vertikalen von den Personen P weggeneigt. Das Pflegepersonal im Observationsraum 20 kann die Personen P auch bei dieser Geometrie des Beobachtungsfensters 11 gut sehen.

In der Fig. 1A ist anhand von Pfeilen dargestellt, was für die Personen P jeweils sichtbar ist. Die geneigten Beobachtungsfenster 11 reflektieren das jeweilige eigene Spiegelbild nicht zur Person P zurück.

Durch einen Einsatz von Polarisationsfolien an beiden Beobachtungsfenstern 11 (jeweils gegeneinander um 90° verdreht) wird eine Komplettdurchsicht durch den Observationsraum 20 verhindert. In Fig. 1A ist auch durch Pfeile dargestellt, dass die Personen P jeweils nur durch ein Beobachtungsfenster 11 geschaut werden kann.

In Fig. 2 ist ein Raum 10 dargestellt, in dem eine Person P in einem Bett 12 flach liegt. Die Blickrichtung B ist etwa 45° zur Ebene des Bettes 12 geneigt, so dass sie auf die erste Anzeigefläche 4 der optischen Anzeigevorrichtung 3 trifft. Dabei ist in der horizontalen ersten Anzeigefläche 4 ein erstes Leuchtmittel 30 (hier ein Leuchtenfeld mit LEDs) angeordnet, das besonders geeignet und ausgebildet ist, eine Beleuchtung dem Tagesverlauf anzupassen. Dies wird noch näher beschrieben werden. Dieses erste Leuchtmittel 30 als Teil der optischen Anzeigevorrichtung 3 ist in einem Blickkegel von 30° angeordnet für die flach liegende Person P angeordnet.

Die beiden Anzeigeflächen 4, 5 der optischen Anzeigevorrichtung 3 sind ferner so gestaltet, dass sie in dieser Lage im Wesentlichen voll im Gesichtsfeld der Person P liegt. Der Blickkegel hat hier einen Öffnungswinkel von 70°, d.h. 35° oberhalb und unterhalb der Blickrichtung B. In dieser Darstellung ist auch erkennbar, dass die optische Anzeigevorrichtung 3 über den Kopf der Person P nach hinten verlängert ist, so dass auch bei einem peripheren Blick die optische Anzeigevorrichtung 3 erfasst wird

In Fig. 3 ist die gleiche Situation dargestellt, wie in Fig. 2, nur dass die Person P im Kopfbereich um 35 bis 40° geneigt ist. Damit verschiebt sich auch die Blickrichtung B entsprechend um 25° nach unten, so dass der Blickkegel von 70° auch Teile des Raums 10 erfasst, die nicht von der optischen Anzeigevorrichtungen 3 bedeckt sind.

In alternativen Ausführungsformen kann die Größe und / oder Form der optischen Anzeigevorrichtung 3 anders ausgebildet sein. So kann statt mehreren ebenen Anzeigeflächen 4, 5, eine gekrümmte Fläche verwendet werden.

In Fig. 4 ist eine Schnittansicht durch ein Detail einer Ausführungsform der optischen Anzeigevorrichtung 3 dargestellt. Ein erstes Leuchtmittel 30 mit LEDs stellt ein Weißlicht zur Verfügung, das einer Grundbeleuchtung, einer Arbeitsbeleuchtung und einer bio-dynamische Beleuchtung dient. In der Ausführung wird hier ein LED GRID mit Weißlicht LEDs mit Beleuchtungsstärken bis min 1700lx verwendet, wobei abwechselnd warmweiß und kaltweiß zusammengeschaltet werden und die stufenlos regelbar sind, so dass im Ergebnis eine Lichttemperatur 2700 bis 6500K erreicht wird und verschiedene Szenarien mit unterschiedlichen Anteilen (Farbtemperatur) für die Person P darstellbar sind.

Ein zweites Leuchtmittel 31 in Form eines LED Grids dient der Anzeige optischer Reize wie optische Muster oder Bilder auf eine Projektionsfläche 32. Das zweite Leuchtmittel 31 bildet die Projektionsebene für das Unterhaltungslicht / die Bespielung mit visuellen Inhalten und / oder für einen RGB Farbraum.

Die Leuchtmittel 30, 31 projizieren auf die lichtdurchscheinende, schallweiche (d.h. ein Material mit einer niedrigen Impedanz) Projektionsfläche 32, so dass die Person P die Muster und / oder Bilder auf der Projektionsfläche 32 wahrnehmen kann. Die Projektionsfläche 32 weist hier einen Lichttransmissionsgrad von 52% auf.

Die beiden Leuchtmittel 30, 31 sind unabhängig voneinander steuerbar, so dass insbesondere technisch unterschiedliche Grids (LED oder OLED) versetzt zueinander, übereinander, gleichzeitig oder unabhängig voneinander ansteuerbare Lichtquellen bilden. Damit werden vollkommen unterschiedliche Qualitäten und Ausführungen innerhalb einer Einrichtung integriert, so dass ein Eindruck einer rahmenlosen architektonisch raumbildend wirksamen Oberfläche (leuchtende Decke und/oder Wand) entsteht, und nicht der eines technischen Bildschirms.

Alternativ können anstelle der LED auch OLED oder andere Leuchtmittel verwendet werden.

An den Rändern der optischen Anzeigevorrichtung 3 ist hier jeweils eine indirekte Beleuchtungsvorrichtung 8 angebracht. Dazu strahlt eine drittes Leuchtmittel 34 auf eine geneigte reflektierende Fläche 35. Die Neigung ist so ausgerichtet, dass genau der Bereich der Kontrastlinie zwischen Decke und optischer Anzeigevorrichtung 3 bestrahlt wird. Damit wird der Randbereich optisch kaschiert, indem eine indirekte Deckenbeleuchtung 8 eingesetzt wird. Dadurch ist für die Person P der Rand der optischen Anzeigevorrichtung 3 visuell aufgelöst, was für die Gesamtbetrachtung der optischen Anzeigevorrichtung 3 aus der stationären Lage der Person P angenehm erscheint, da es die Blendung der Person P reduziert. Grundsätzlich ist es möglich, dass nicht alle Seiten der optischen Anzeigevorrichtung 3 mit einer indirekten Beleuchtung 8 versehen werden.

In Fig. 4A ist eine schematische perspektivische Ansicht der optischen Anzeigevorrichtung 3 dargestellt. Dabei erstreckt sich hier erste Leuchtmittel 30 nur über einen Teil des zweiten Leuchtmittels 31, wobei das zweite Leuchtmittel 31 hier so geformt ist, das schließlich die ersten und zweite Anzeigefläche 3, 4 gebildet werden.

In Fig. 5 ist eine Ausführungsform einer Schallschutzvorrichtung 2 dargestellt, die typischerweise am Kopfende der Person P (siehe Fig. 1) angeordnet ist. Diese schirmt akustisch aktive medizinische Geräte 1 gegenüber dem Raum 10, insbesondere aber auch gegenüber der Person P ab. An der Seite und hinter der Schallschutzvorrichtung 2 sind Anschlüsse für Elektrizität, Datenleitungen, Sauerstoff und / oder Druckluft angeordnet. In diese Schallschutzvorrichtung 2 ist ebenfalls ein Monitor für physiologische Daten (hämodynamischer Monitor) der Person P integriert.

In Fig. 6 ist eine Ausführungsform einer Schallschutzbarriere 7 dargestellt, wie sie z.B. zwischen zwei Personen P in einem Raum 10 angeordnet werden kann. Damit werden die Personen P gegenseitig vor Emissionen geschützt. An der Schallschutzbarriere 7 kann ein Bereich 9 zur Anbringung persönlicher Gegenstände angeordnet sein, der bei einem seitlichen Blick der Person P wahrgenommen werden kann. Ansonsten sind in der Schallschutzbarriere 7 bewegliche Wagen angebracht, die z.B. bei der Pflege oder der Therapie der Person P benötigt werden.

Des Weiteren können in der Schallschutzbarriere 7 und / oder auch in der Schallschutzvorrichtung mindestens zwei Öffnungen angeordnet sein, durch die Schall aus dem Raum 10 eintreten kann. Im Inneren der Schallschutzbarriere 7 und / oder der Schallschutzvorrichtung 2 ist Dämpfungsmaterial angeordnet, so dass die Schallenergie aus dem Raum absorbiert wird.

### Bezugszeichenliste

- 1: akustische aktive Geräte
- 2: Schallschutzvorrichtung
- 3: optische Anzeigevorrichtung
- 4: erste Anzeigefläche der optischen Anzeigevorrichtung
- 5: zweite Anzeigefläche der optischen Anzeigevorrichtung
- 6: Lichtsteuerung
- 7: Schallschutzbarriere
- 8: indirekte Deckenbeleuchtung
- 9: Bereich zur Anbringung persönlicher Gegenstände
- 10: Raum
- 11: Beobachtungsfenster
- 12: Bett

- 20: schallgeschützter Raum (z.B. Observationsraum)

- 30: erstes Leuchtmittel, Leuchtenfeld
- 31: zweites Leuchtmittel, Leuchtenfeld
- 32: Bespannung / Projektionsfläche
- 34: drittes Leuchtmittel
- 35: reflektierende Fläche

- B: Blickrichtung
- E: Ebene
- P: Person

- α: Blickwinkel
- β: Winkel zwischen zwei Anzeigenflächen der optischen Anzeigevorrichtung

## Patentansprüche

1. System zur Beeinflussung der Sinne einer Person in einem Raum zur Messung und / oder Beeinflussung physiologischer Parameter der Person, wobei die Person während der Messung und / oder Beeinflussung im Wesentlichen stationär liegend im Raum ist, aufweisend
eine Schallschutzvorrichtung (2) zur Abschirmung akustisch aktiver Geräte (1) gegenüber der Person (P), wobei die akustisch aktiven Geräte (1) zur Messung und / oder Beeinflussung der physiologischen Parameter im Raum (10) dienen,
und
eine optische Anzeigevorrichtung (3) zur Anbietung optischer Reize und / oder Signale für die Person (P) im Gesichtsfeld der Person (P), wobei sich die optische Anzeigevorrichtung (3) in mindestens einer Blickrichtung (B) der stationär liegenden Person (P) über einen Blickwinkel (α) von mindestens 40° und maximal 180°, insbesondere zwischen 55° und 75° ganz insbesondere 70° erstreckt, wobei sich der Blickwinkel (α) in einer Ebene erstreckt, die sich von der Blickrichtung (B) von oben nach unten oder von der Blickrichtung nach links und rechts erstreckt,
**gekennzeichnet durch**
die optische Anzeigevorrichtung (3) umfassend ein erstes Leuchtmittel (30), insbesondere ein Leuchtenfeld mit LEDs oder ein OLED, zur Funktionsbeleuchtung, Arbeitsbeleuchtung und / oder biodynamischen Beleuchtung des Raums (10) im Bereichs der Person (P) und
ein vom ersten Leuchtmittel (30) unabhängig steuerbares zweites Leuchtmittel (31) der optischen Anzeigevorrichtung (3), insbesondere ein LED Grid für die Anzeige von Mustern oder optischen Reizen, insbesondere als Unterhaltungslicht oder zur Bespielung mit visuellen Inhalten,
wobei das erste und zweite Leuchtmittel (31, 32) übereinander angeordnet sind, wobei die überlagerten optischen Signale der beiden Leuchtmittel (31, 32) auf eine transluzente Projektionsfläche (32) der optischen Anzeigevorrichtung (3) treffen, die sich im Blickfeld der Person (P) befindet.

2. System nach Anspruch 1, **dadurch gekennzeichnet, dass** insbesondere die Schallschutzvorrichtung (2) die mittlere Schallemission der aktiven akustischen Geräte (1) an der Person (P) im Raum (10) auf höchstens 55 dB(A), insbesondere höchstens 40 dB(A) reduziert.

3. System nach Anspruch 1 oder 2, **dadurch gekennzeichnet, dass** Schallschutzvorrichtung (2) ein Mittel zum Auffangen und Dämpfen von innerhalb der Schallschutzvorrichtung (2) abgestrahltem und / oder vom Raum (10) in die Schallschutzvorrichtung (2) eingestrahltem Schall aufweist.

4. System nach mindestens einem der vorhergehenden Ansprüche, **dadurch gekennzeichnet, dass** die optische Anzeigevorrichtung (3) sich bei der stationär liegenden Person (P) über die Kopfposition hinaus nach hinten erstreckt.

5. System nach mindestens einem der vorhergehenden Ansprüche, **dadurch gekennzeichnet, dass** die optische Anzeigevorrichtung (3) mindestens zwei räumlich unterschiedlich orientierte Anzeigeflächen (4, 5) oder eine gekrümmte Anzeigefläche aufweist, wobei die optische Anzeigevorrichtung (3) mindestens teilweise oberhalb der stationär liegenden Person (P) in einer Ebene (E) angeordnet ist, die insbesondere im Wesentlichen parallel zu der liegenden Position der Person (P) orientiert ist.

6. System nach mindestens einem der vorhergehenden Ansprüche, **dadurch gekennzeichnet, dass** eine zweiter Anzeigefläche (5) der optischen Anzeigevorrichtung (3) in Blickrichtung (B) der Person (P) unter einem Winkel (β) zwischen 90° und 160°, insbesondere unter einem Winkel (β) zwischen 110° und 120°, ganz insbesondere mit einem Winkel (β) von 114° angeordnet ist, wobei der Winkel (β) von einer Ebene (E) gemessen wird, die sich oberhalb und parallel zu der stationär liegenden Person (P) erstreckt.

7. System nach mindestens einem der vorhergehenden Anspruch, **dadurch gekennzeichnet, dass** ein Übergangsbereich zwischen der ersten Anzeigefläche (4) der optischen Anzeigevorrichtung (3) und der zweiten Anzeigefläche (5) der optischen Anzeigevorrichtung (3) gerundet ausgebildet ist, insbesondere mit einem Krümmungsradius zwischen 20 und 40 cm.

8. System nach mindestens einem der vorhergehenden Ansprüche, **dadurch gekennzeichnet, dass** die optischen Reize und / oder Signale ausgehend von der optischen Anzeigevorrichtung (3) Informationsdarstellungen, eine Arbeitsbeleuchtung und / oder einer biodynamische Beleuchtung umfassen.

9. System nach mindestens einem der vorhergehenden Ansprüche, **dadurch gekennzeichnet, dass** mit der optischen Anzeigevorrichtung (3) optische Reize und / oder Signale für die stationär liegende Person (P) anzeigbar sind, wobei die Reize und / oder Signale vorprogrammiert und / oder durch an der Person (P) gemessene Daten steuerbar sind.

10. Raumausstattung mit einem System zur Beeinflussung der Sinne einer Person in einem Raum zur Messung und / oder Beeinflussung physiologischer Parameter der Person, wobei die Person während der Messung und / oder Beeinflussung im Wesentlichen stationär liegend im Raum ist, aufweisend schalldämmende Materialien in mindestens Teilen des Bodens, den Wänden und / oder der Decke des Raumes (10), wobei das System folgendes umfasst
eine Schallschutzvorrichtung (2) zur Abschirmung akustisch aktiver Geräte (1) gegenüber der Person (P), wobei die akustisch aktiven Geräte (1) zur Messung und / oder Beeinflussung der physiologischen Parameter im Raum (10) dienen,
eine optische Anzeigevorrichtung (3) zur Anbietung optischer Reize und / oder Signale für die Person (P) im Gesichtsfeld der Person (P), wobei sich die optische Anzeigevorrichtung (3) in mindestens einer Blickrichtung (B) der stationär liegenden Person (P) über einen Blickwinkel (α) von mindestens 40° und maximal 180°, insbesondere zwischen 55° und 75° ganz insbesondere 70° erstreckt, wobei sich der Blickwinkel (α) in einer Ebene erstreckt, die sich von der Blickrichtung (B) von oben nach unten oder von der Blickrichtung nach links und rechts erstreckt,
die optische Anzeigevorrichtung (3) umfassend ein erstes Leuchtmittel (30), insbesondere ein Leuchtenfeld mit LEDs oder ein OLED, zur Funktionsbeleuchtung, Arbeitsbeleuchtung und / oder biodynamischen Beleuchtung des Raums (10) im Bereichs der Person (P) und
ein vom ersten Leuchtmittel (30) unabhängig steuerbares zweites Leuchtmittel (31) der optischen Anzeigevorrichtung (3), insbesondere ein LED Grid für die Anzeige von Mustern oder optischen Reizen, insbesondere als Unterhaltungslicht oder zu Bespielung mit visuellen Inhalten,
wobei das erste und zweite Leuchtmittel (31, 32) übereinander angeordnet sind, wobei die überlagerten optischen Signale der beiden Leuchtmittel (31, 32) auf eine transluzente Projektionsfläche (32) der optischen Anzeigevorrichtung (3) treffen, die sich im Blickfeld der Person (P) befindet.

11. Raumausstattung nach Anspruch 10 mit mindestens zwei Systemen nach einem der Ansprüche 1 bis 9, wobei zwischen zwei stationär liegenden Personen (P) jeweils eine Schallschutzbarriere (7), insbesondere als Sichtschutzbarriere angeordnet ist, wobei die Schallschutzbarriere (7) insbesondere mindestens teilweise eine Oberfläche mit einer Holzstruktur aufweist.

12. Raumausstattung nach Anspruch 10 oder 11, **gekennzeichnet durch** mindestens ein Beobachtungsfenster (11) für Beobachter außerhalb des Raums (10), wobei das mindestens eine Beobachtungsfenster (11) zur Vermeidung von Spiegelbildern der liegenden stationären Person (P) gegenüber der Wand des Raumes (10) um 5 bis 10°, insbesondere 8° geneigt ist.

13. Raumausstattung nach mindestens einem der Ansprüche 10 bis 12, **dadurch gekennzeichnet, dass** zwei gegenübergelegene Räume (10) über einen dazwischengeschaltetem Observationsraum (20) mit mindestens zwei Beobachtungsfenstern (11) verbunden sind, so dass eine Beobachtung von zwei Räumen (10) aus dem Observationsraum (20) ermöglicht wird.

14. Raumausstattung nach mindestens einem der Ansprüche 10 bis 13, **gekennzeichnet durch** eine Lichtsteuerung (6) des Raums (10) mit folgenden Lichtparametern :
• Lichtstärke tags: 300 lx (morgens), 1.700 lx (mittags) und 100 lx (abends),
• Lichtstärke nachts <3 lx,
• Lichtfarbe: 2700-6500 K im Tagesverlauf angepasst an den natürlichen Verlauf,
• Lichttemperatur stufenlos regelbar in verschiedenen Szenarien mit unterschiedlichen prozentualen Anteilen und / oder
• Leuchtdichte <500 cd m⁻².

15. Raumausstattung nach mindestens einem der Ansprüche 10 bis 14, **gekennzeichnet durch** einem schallgeschützten Raum (20) mit mindestens einem Arbeitsplatz für Betreuungspersonal, einer Alarmstation zur Wiedergabe akustischer Alarme, einer Glasabwurfvorrichtung, einem Wäschelager und / oder einem Medikamentenlager.

## Claims

1. System for influencing the senses of a person in a room for measuring and/or influencing physiological parameters of the person, wherein the person is substantially stationary and lying in the room during the measurement and/or influencing, having
a sound insulating apparatus (2) for screening off acoustically active devices (1) with respect to the person (P), wherein the acoustically active devices (1) serve for measuring and/or influencing the physiological parameters in the room (10), and
an optical display apparatus (3) for offering optical stimuli and/or signals for the person (P) in the field of view of the person (P), wherein the optical display apparatus (3) extends in at least one viewing direction (B) of the stationary lying person (P) over a viewing angle (α) of at least 40° and at most 180°, in particular between 55° and 75°, very particularly of 70°, wherein the viewing angle (α) extends in a plane that extends down from the viewing direction (B) or to the left and to the right from the viewing direction,
**characterized by**
the optical display apparatus (3) comprising a first luminous means (30), in particular a luminous field having LEDs or an OLED, for functional illumination, task lighting and /or biodynamic lighting of the room (10) within the region of the person (P), and
a second luminous means (31) of the optical display apparatus (3), which is controllable independently of the first luminous means (30), in particular an LED grid for displaying patterns or optical stimuli, in particular as an entertainment light or for playing visual contents,
wherein the first and second luminous means (31, 32) are arranged one above the other, wherein the superposed optical signals of the two luminous means (31, 32) are incident on a translucent projection area (32) of the optical display apparatus (3), which is located in the field of view of the person (P).

2. System according to Claim 1, **characterized in that** in particular the sound insulating apparatus (2) reduces the average sound emission of the active acoustic devices (1) at the person (P) in the room (10) to at most 55 dB(A), in particular at most 40 dB(A).

3. System according to Claims 1 or 2, **characterized in that** the sound insulating apparatus (2) has a means for absorbing and damping sound that is emitted within the sound insulating apparatus (2) and/or directed from the room (10) into the sound insulating apparatus (2).

4. System according to at least one of the preceding claims, **characterized in that** the optical display apparatus (3) extends near the stationary lying person (P) beyond the head position to the rear.

5. System according to at least one of the preceding claims, **characterized in that** the optical display apparatus (3) has at least two spatially differently oriented display areas (4, 5) or a curved display area, wherein the optical display apparatus (3) is arranged at least partially above the stationary lying person (P) in a plane (E) that is oriented in particular substantially parallel to the lying position of the person (P).

6. System according to at least one of the preceding claims, **characterized in that** a second display area (5) of the optical display apparatus (3) is arranged in the viewing direction (B) of the person (P) at an angle (β) of between 90° and 160°, in particular at an angle (β) of between 110° and 120°, very particularly at an angle (β) of 114°, wherein the angle (β) is measured from a plane (E) that extends above and parallel to the stationary lying person (P).

7. System according to at least one of the preceding claims, **characterized in that** a transition region between the first display area (4) of the optical display apparatus (3) and the second display area (5) of the optical display apparatus (3) has a rounded configuration, in particular having a radius of curvature of between 20 and 40 cm.

8. System according to at least one of the preceding claims, **characterized in that** the optical stimuli and/or signals that originate from the optical display apparatus (3) comprise information representations, task lighting and/or biodynamic lighting.

9. System according to at least one of the preceding claims, **characterized in that** optical stimuli and/or signals are displayable for the stationary lying person (P) using the optical display apparatus (3), wherein the stimuli and/or signals are preprogrammed and/or controllable by way of data measured on the person (P).

10. Room setup having a system for influencing the senses of a person in a room for measuring and/or influencing physiological parameters of the person, wherein the person is substantially stationary and lying in the room during the measurement and/or influencing, having sound-damping materials in at least part of the floor, the walls and/or the ceiling of the room (10), wherein the system comprises the following a sound insulating apparatus (2) for screening off acoustically active devices (1) with respect to the person (P), wherein the acoustically active devices (1) serve for measuring and/or influencing the physiological parameters in the room (10),
an optical display apparatus (3) for offering optical stimuli and/or signals for the person (P) in the field of view of the person (P), wherein the optical display apparatus (3) extends in at least one viewing direction (B) of the stationary lying person (P) over a viewing angle (α) of at least 40° and at most 180°, in particular between 55° and 75°, very particularly of 70°, wherein the viewing angle (α) extends in a plane that extends down from the viewing direction (B) or to the left and to the right from the viewing direction,
the optical display apparatus (3) comprising a first luminous means (30), in particular a luminous field having LEDs or an OLED, for functional illumination, task lighting and /or biodynamic lighting of the room (10) within the region of the person (P), and
a second luminous means (31) of the optical display apparatus (3), which is controllable independently of the first luminous means (30), in particular an LED grid for displaying patterns or optical stimuli, in particular as an entertainment light or for playing visual contents,
wherein the first and second luminous means (31, 32) are arranged one above the other, wherein the superposed optical signals of the two luminous means (31, 32) are incident on a translucent projection area (32) of the optical display apparatus (3), which is located in the field of view of the person (P).

11. Room setup according to Claim 10 having at least two systems according to one of Claims 1 to 9, wherein in each case one sound insulating barrier (7) is arranged between two stationary lying persons (P), in particular as a viewing protection barrier, wherein the sound insulating barrier (7) in particular has at least partially a surface having a wood structure.

12. Room setup according to Claim 10 or 11, **characterized by** at least one observation window (11) for observers outside the room (10), wherein the at least one observation window (11) is inclined by 5 to 10°, in particular 8°, with respect to the wall of the room (10) to avoid reflected images of the lying stationary person (P).

13. Room setup according to at least one of Claims 10 to 12, **characterized in that** two opposite rooms (10) are connected via an intermediate observation room (20) having at least two observation windows (11), with the result that observation of two rooms (10) from the observation room (20) is made possible.

14. Room setup according to at least one of Claims 10 to 13, **characterized by** a light controller (6) of the room (10) having the following light parameters:
• light intensity during the day: 300 lx (in the morning), 1700 lx (noon) and 100 lx (in the evening),
• light intensity at night: <3 lx,
• light color: 2700-6500 K over the course of the day matched to the natural progression,
• light temperature infinitely variable in different scenarios with different percentage proportions, and/or
• luminance <500 cd m⁻².

15. Room setup according to at least one of Claims 10 to 14, **characterized by** a soundproofed room (20) having at least one workstation for care providers, an alarm station for playing acoustic alarms, a glass discarding apparatus, a linen storage means and/or a pharmaceutical storage means.

## Revendications

1. Système, destiné à influencer les sens d'une personne dans une pièce pour mesurer et/ou influencer des paramètres physiologiques de la personne, pendant la mesure et/ou l'influence, la personne étant couchée de manière sensiblement stationnaire dans la pièce, comportant
un dispositif insonorisant (2) destiné à blinder des appareils acoustiquement actifs (1) par rapport à la personne (P), les appareils acoustiquement actifs (1) servant à mesurer et/ou à influencer les paramètres physiologiques dans la pièce (10),
et
un dispositif d'affichage optique (3) destiné à proposer des stimulations et/ou signaux optiques pour la personne (P) dans le champ visuel de la personne (P), dans au moins une direction de visée (B) de la personne (P) couchée de manière stationnaire, le dispositif d'affichage optique (3) s'étendant sur un angle de visée (α) d'au moins 40° et d'un maximum de 180°, particulièrement compris entre 55° et 75°, très particulièrement de 70°, l'angle de visée (α) s'étendant dans un plan, qui s'étend à partir de la direction de visée (B) du haut vers le bas ou à partir de la direction de visée vers la gauche et la droite,
**caractérisé par**
le dispositif d'affichage optique (3) comprenant un premier moyen lumineux (30), particulièrement une zone lumineuse pourvue de LED ou d'une OLED, pour l'éclairage fonctionnel, l'éclairage opérationnel et/ou l'éclairage biodynamique de la pièce (10) dans la zone de la personne (P) et
un deuxième moyen lumineux (31) du dispositif d'affichage optique (3), susceptible d'être commandé indépendamment du premier moyen lumineux (30), particulièrement une grille de LED, destinée à afficher des motifs ou des stimulations optiques, particulièrement sous la forme d'une lumière d'ambiance ou pour diffuser des contenus optiques,
les premier et deuxième moyens lumineux (31, 32) étant placés en superposition, les signaux optiques superposés des deux moyens lumineux (31, 32) étant incidents sur une surface de projection (32) translucide du dispositif d'affichage optique (3) qui se trouve dans le champ de vision de la personne (P).

2. Système selon la revendication 1, **caractérisé en ce que** particulièrement le dispositif insonorisant (2) réduit l'émission sonore moyenne des appareils acoustiquement actifs (1) restituée à la personne (P) dans la pièce (10) à un maximum de 55 dB (A), particulièrement à un maximum de 40 dB(A).

3. Système selon la revendication 1 ou 2, **caractérisé en ce que** le dispositif insonorisant (2) comporte un moyenne destiné à capturer et à amortir du bruit émis à l'intérieur du dispositif insonorisant (2) et/ou émis à partir de la pièce (10) dans le dispositif insonorisant (2).

4. Système selon au moins l'une quelconque des revendications précédentes, **caractérisé en ce que** près de la personne (P) couchée de manière stationnaire, le dispositif d'affichage optique (3) s'étend vers l'arrière, au-delà de la position de la tête.

5. Système selon au moins l'une quelconque des revendications précédentes, **caractérisé en ce que** le dispositif d'affichage optique (3) comporte au moins deux surfaces d'affichage (4, 5) à orientation différente dans l'espace ou une surface d'affichage curviligne, le dispositif d'affichage optique (3) étant placé au moins en partie au-dessus de la personne (P) couchée de manière stationnaire dans un plan (E) qui est particulièrement orienté sensiblement à la parallèle de la position couchée de la personne (P) .

6. Système selon au moins l'une quelconque des revendications précédentes, **caractérisé en ce que** dans la direction de visée (B) de la personne (P), une deuxième surface d'affichage (5) du dispositif d'affichage optique (3) est disposée sous un angle (β) compris entre 90° et 160°, particulièrement sous un angle (β) compris entre 110° et 120°, tout particulièrement sous un angle (β) de 114°, l'angle (β) étant mesuré à partir d'un plan (E) s'étendant au-dessus et à la parallèle de la personne (P) couchée de manière stationnaire.

7. Système selon au moins l'une quelconque des revendications précédentes, **caractérisé en ce qu'**une zone transitoire entre la première surface d'affichage (4) du dispositif d'affichage optique (3) et la deuxième surface d'affichage (5) du dispositif d'affichage optique (3) est conçue de forme arrondie, particulièrement avec un rayon de courbure compris entre 20 et 40 cm.

8. Système selon au moins l'une quelconque des revendications précédentes, **caractérisé en ce que** les stimulations et/ou signaux optiques comprennent à partir du dispositif d'affichage optique (3) des représentations d'informations, un éclairage opératoire et/ou un éclairage biodynamique.

9. Système selon au moins l'une quelconque des revendications précédentes, **caractérisé en ce que** le dispositif d'affichage optique (3) permet d'afficher des stimulations et/ou signaux optiques à l'attention de la personne (P) couchée de manière stationnaire, les stimulations et/ ou signaux étant préprogrammés et/ou susceptibles d'être commandés par des données mesurées sur la personne (P).

10. Équipement d'une pièce avec un système, destiné à influencer les sens d'une personne dans une pièce pour mesurer et/ou influencer des paramètres physiologiques de la personne, pendant la mesure et/ou l'influence, la personne étant couchée de manière sensiblement stationnaire dans la pièce, comportant des matières insonorisantes dans au moins des parties du sol, dans les murs et /ou dans le plafond de la pièce (10), le système comprenant ce qui suit
un dispositif insonorisant (2) destiné à blinder des appareils acoustiquement actifs (1) par rapport à la personne (P), les appareils acoustiquement actifs (1) servant à mesurer et/ou à influencer les paramètres physiologiques dans la pièce (10),
un dispositif d'affichage optique (3) destiné à proposer des stimulations et/ou signaux optiques pour la personne (P) dans le champ visuel de la personne (P), dans au moins une direction de visée (B) de la personne (P) couchée de manière stationnaire, le dispositif d'affichage optique (3) s'étendant sur un angle de visée (α) d'au moins 40° et d'un maximum de 180°, particulièrement compris entre 55° et 75°, très particulièrement de 70°, l'angle de visée (α) s'étendant dans un plan, qui s'étend à partir de la direction de visée (B) du haut vers le bas ou à partir de la direction de visée vers la gauche et la droite,
le dispositif d'affichage optique (3) comprenant un premier moyen lumineux (30), particulièrement une zone lumineuse pourvue de LED ou d'une OLED, pour l'éclairage fonctionnel, l'éclairage opérationnel et/ou l'éclairage biodynamique de la pièce (10) dans la zone de la personne (P) et
un deuxième moyen lumineux (31) du dispositif d'affichage optique (3), susceptible d'être commandé indépendamment du premier moyen lumineux (30), particulièrement une grille de LED, destinée à afficher des motifs ou des stimulations optiques, particulièrement sous la forme d'une lumière d'ambiance ou pour diffuser des contenus optiques,
les premier et deuxième moyens lumineux (31, 32) étant placés en superposition,
les signaux optiques superposés des deux moyens lumineux (31, 32) étant incidents sur une surface de projection (32) translucide du dispositif d'affichage optique (3) qui se trouve dans le champ de vision de la personne (P).

11. Équipement d'une pièce selon la revendication 10 avec au moins deux systèmes selon l'une quelconque des revendications 1 à 9, entre deux personnes (P) couchées de manière stationnaire étant placée chaque fois une barrière insonorisante (7), particulièrement sous la forme d'une barrière de protection visuelle, la barrière insonorisante (7) comportant particulièrement au moins en partie une surface dotée d'une structure en bois.

12. Équipement d'une pièce selon la revendication 10 ou 11, **caractérisé par** au moins une fenêtre d'observation (11) pour des observateurs situés à l'extérieur de la pièce (10), pour éviter des reflets de la personne (P) couchée de manière stationnaire, l'au moins une fenêtre d'observation (11) étant inclinée de la valeur de 5 à 10°, particulièrement de 8° par rapport au mur de la pièce (10).

13. Équipement d'une pièce selon au moins l'une quelconque des revendications 10 à 12, **caractérisé en ce que** deux pièces (10) placées en vis-à-vis sont reliées par l'intermédiaire d'une pièce d'observation (20) interposée avec au moins deux fenêtres d'observation (11), de sorte à permettre une observation de deux pièces (10) à partir de la pièce d'observation (20).

14. Équipement d'une pièce selon au moins l'une quelconque des revendications 10 à 13, **caractérisé par** une commande de la lumière (6) de la pièce (10) avec les paramètres lumineux suivants :
• Intensité lumineuse diurne : 300 lx (le matin), 1.700 lx (à midi) et 100 lx (le soir),
• Intensité lumineuse nocturne < 3 lx,
• Couleur de la lumière : 2700 à 6500 K au fil du jour, adaptée à l'évolution naturelle,
• Température de la lumière à réglage continu dans différents scénarios avec différentes parts en pourcentage et/ou
• Luminance < 500 cd m⁻².

15. Équipement d'une pièce selon au moins l'une quelconque des revendications 10 à 14, **caractérisé par** une pièce (20) insonorisée dotée d'au moins un poste de travail pour du personnel d'assistance, un poste d'alerte, destiné à reproduire des alertes acoustiques, un dispositif pour jeter le verre, un stock de linge et/ou un stock de médicaments.
